# EUROPÄISCHE PATENTSCHRIFT

(11) **EP 0 859 595 B1**
(45) Veröffentlichungstag und Bekanntmachung des Hinweises auf die Patenterteilung: **03.11.1999**
(21) Anmeldenummer: 96931820.3
(22) Anmeldetag: 21.09.1996
(51) Int. Cl.: A61K 9/06, A61K 9/70

(54) **THERAPEUTISCHE ZUBEREITUNG ZUR TRANSDERMALEN APPLIKATION VON WIRKSTOFFEN DURCH DIE HAUT**
THERAPEUTIC PREPARATION FOR THE TRANSDERMAL ADMINISTRATION OF ACTIVE SUBSTANCES
PREPARATION THERAPEUTIQUE POUR L'ADMINISTRATION TRANSDERMIQUE DE SUBSTANCES ACTIVES

(30) Priorität: 06.11.1995 DE 19541260
(43) Veröffentlichungstag der Anmeldung: 26.08.1998
(73) Patentinhaber: LTS LOHMANN Therapie-Systeme GmbH, 56567 Neuwied (DE)
(72) Erfinder: ASMUSSEN, Bodo, D-56170 Bendorf-Sayn (DE); KOCH, Andreas, D-56567 Neuwied (DE); MATUSCH, Rudolf, D-35041 Marburg (DE)
(74) Vertreter: Flaccus, Rolf-Dieter, Dr.
(86) Internationale Anmeldenummer: EP9604138
(87) Internationale Veröffentlichungsnummer: WO9717061

(56) Entgegenhaltungen:
- WO-A-94/21230
- CHEMICAL ABSTRACTS, vol. 124, no. 22, 27.Mai 1996 Columbus, Ohio, US; abstract no. 298562, XP002024379 & J. PHARM. SCI., Bd. 85, Nr. 6, 1996, Seiten 643-648, J.C. TSAI ET AL.: "METABOLIC APPROACHES TO ENHANCE TRANSDERMAL DRUG DELIVERY.1.EFFECT OF LIPID SYNTHESIS INHIBITORS"

## Beschreibung

Die Erfindung betrifft eine therapeutische Zubereitung zur transdermalen Applikation von Wirkstoffen durch die Haut, enthaltend eine Steigerung der perkutanen Absorptionsrate von transdermal normalerweise nur ungenügend resorbierbaren Wirkstoffen bewirkende Zusätze.

Eines der wesentlichsten Probleme bei Darreichungsformen mit perkutan resorbierbaren Wirkstoffen, z.B. bei transdermalen therapeutischen Systemen, stellt die Überwindung der natürlichen Permeabilitätsbarriere der Haut dar. Diese dient der epidermalen Schutzfunktion der Haut und wird durch breite lamellierte Lipid-Doppelschichten in den Interzellularräumen der Epidermis gebildet und setzt perkutanen Resorptionsversuchen als Barriere Widerstand entgegen.

Um hier Abhilfe zu schaffen, werden schon seit längerer Zeit therapeutischen Zubereitungen zur transdermalen Applikation von Wirkstoffen permeationsfördernde Zusätze, sog. klassische Enhancer zugesetzt, welche die perkutane Absorptionsrate von lipophilen oder hydrophilen Arzneiwirkstoffen über einen längeren Zeitraum erhöhen. Jedoch hat sich herausgestellt, daß eine Anzahl von Wirkstoffen dennoch eine ungenügende perkutane Absorptionsrate aufweisen. Gelegentlich wurde von der Maßnahme Gebrauch gemacht, die Fläche einer transdermalen therapeutischen Zubereitung vergleichsweise zu vergrößern, um dadurch einen therapeutisch wirksamen Wirkstofffluß durch die Haut in den Organismus zu erreichen. Aber auch hiermit ergehen sich eine Reihe von Nachteilen, weil einerseits ein derartiges Pflaster unnötig teuer und aufwendig in der Herstellung wird, und weil andererseits ein großes transdermales Pflaster vom Patienten als störend empfunden wird. Bei Überdeckung eines größeren Hautareals kann es durch Muskelspiel oder andere Körperbewegungen leicht zu teilweisen Ablösungen des Pflasters kommen, wodurch dann der geregelte Wirkstofffluß erheblich gestört wird.

Die Wirkungsweise von klassischen Enhancern ist bis ins letzte Detail noch nicht restlos erforscht, jedoch schreibt man den zur Zeit üblich eingesetzten Enhancern physikochemische Wirkmechanismen zu, beispielsweise eine Erhöhung der Lipidlöslichkeit über veränderte Verteilungskoeffizienten des Wirkstoffs in den epidermalen Lipid-Doppelschichten oder Senkung des Diffusionskoeffizienten infolge EntropieAbnahme des flüssigkristallinen Zustandes der Hautlipide durch sterische Effekte und polare Wechselwirkungen zwischen Enhancer und Hautlipid.

Es ist weiter bekannt, daß Hautstrukturen auch dadurch verändert werden können, daß durch direkten Eingriff in biochemische Regenerationsprozesse zum Beispiel in der Epidermis Einfluß auf die Permeabilitätsbarriere der Haut genommen wird. So wird beispielsweise in einer Arbeit von Proksch (J. Hautarzt, 1955, V. 46 N 2, S. 76-80) beschrieben, daß die topische Anwendung des spezifischen HMG-CoA-Inhibitors LOVASTATIN zu einem Sinken des Cholesterolspiegels in der Haut bei gleichzeitigem Anstieg des transepidermalen Wasserverlustes und der DNA-Synthese in der Epidermis führt; die natürliche Permeabilitätsbarriere der Haut wird dadurch gestört.

WO 94/21230 beschreibt Zusammensetzungen zur topischen Anwendung, enthaltend einen HMG-CoA-Reduktase-Inhibitor wie z.B. Lovastatin zur Steigerung der Absorptionsrate von Wirkstoffen. Bevorzugte Wirkstoff konzentrationen liegen im Bereich von 0.01 - 5%. Transdermale Therapeutische Systeme werden in diesem Dokument nicht erwähnt.

In der DE 36 34 016 ist die Verwendung von Lipidsenkern in transdermalen therapeutischen Systemen erwähnt. Die dort erfolgte Benennung von Lipidsenkern in Kombination mit anderen Wirkstoffen dient jedoch ausschließlich therapeutischen Zielsetzungen, während eine Steigerung der perkutanen Absorptionsrate von transdermal normalerweise nur ungenügend resorbierbaren Wirkstoffen nicht gedacht ist.

Der Erfindung liegt die Aufgabe zugrunde, eine therapeutische Zubereitung der im Oberbegriff von Anspruch 1 genannten Art weiterzuentwickeln und soweit zu verbessern, daß mit dieser die Hautstruktur dahingehend verändert wird, daß eine erhöhte Resorption von Arzneiwirkstoffen infolge eines verringerten Diffusionswiderstandes der Haut, speziell der Epidermis, erreicht wird, sodaß auch normalerweise ungenügend resorbierbare Wirkstoffe eine wesentlich verbesserte Permeationsrate aufweisen.

Zur Lösung der Aufgabe wird mit der Erfindung vorgeschlagen, daß die Zusätze zur Steigerung der perkutanen Absorptionsrate einer Zubereitung HMG-CoA-Reduktase-Inhibitoren sind.
Damit wird der in der Arbeit von Proksch erwähnte Effekt einer Störung der natürlichen Permeabilitätsbarriere der Haut, die nach dieser Veröffentlichung durch Kombination von Lipidsenkern mit anderen Wirkstoffen ausschließlich therapeutischen Zielsetzungen folgt, gezielt eingesetzt, um durch HMG-CoA-Reduktase-Inhibitoren mit mindestens 0,1 jedoch höchstens 20 Ma% als permeationsfördernde Zusätze die perkutane Absorptionsrate von lipophilen oder hydrophilen Arzneiwirkstoffen über einen längeren Zeitraum zu erhöhen.

Im Gegensatz zu den klassischen Enhancern liegt der Erfindung bezüglich der überraschend erreichten Permeationsförderung ein biochemisches Wirkprinzip zugrunde, welches über eine gestörte Permeabilitätsbarriere in der Epidermis über einen längeren Zeitraum hinweg ein "Fenster" schafft, durch das aufgrund Ihrer physikochemischen Eigenschaften problematische Arzneiwirkstoffe, z.B. mit Molekülmassen über 400 Dalton, hohem Schmelzpunkt, geringer Wasserlöslichkeit bzw. niedrigem Verteilungskoeffizienten, Wasser/Öl, überhaupt erst, und sogar verstärkt, die Hautpassage transdermal überwinden können. Die Unterdrückung der durch HMG-CoA-Reduktase-Inhibitoren bewirkten epidermalen Biolipidsynthese verhindert auch, daß natürliche Reparaturmechanismen der Epidermis zur Wiederherstellung einer gestörten Permeabilitätsbarriere, wie sie bei Einsatz von Enhancern mit fettlöslichen Eigenschaften üblich sind, erfolglos bleiben. Der permeationsfördernde Effekt, das sog. "Fenster" innerhalb der Permeabilitätsbarriere ist daher von längerer Dauer und damit auch von praktischer Relevanz in der transdermalen Therapie von Arzneiwirkstoffen.

Das Problem hautirritierender Nebenwirkungen, die den praktischen Einsatz vieler potentieller Enhancer verhindern und speziell auch für HMG-CoA-Reduktase-Inhibitoren wie z.B. LOVASTATIN zutreffen, wird erfindungsgemäß dadurch relativiert, daß höchstens 20 Ma% der infrage kommenden Lipidsenker in transdermalen therapeutischen Systemen eingesetzt werden.

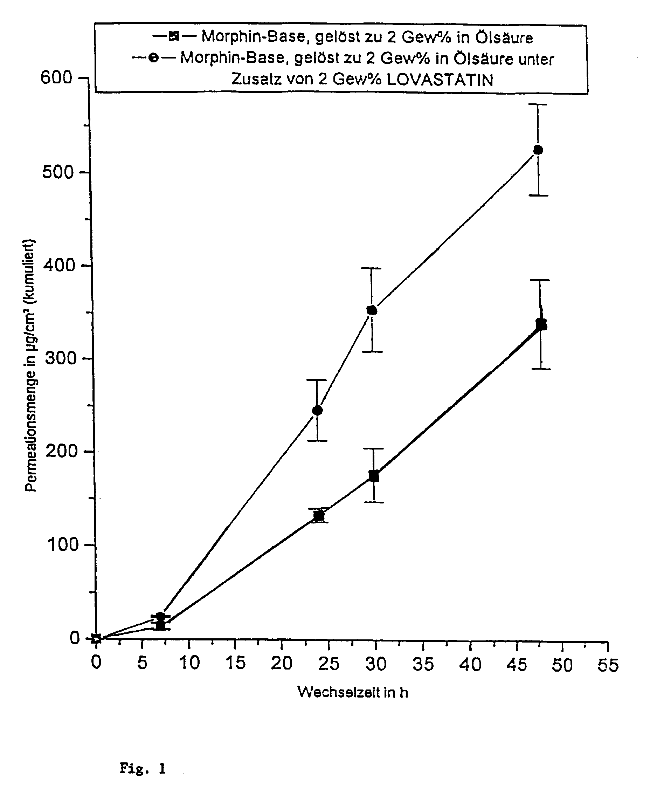

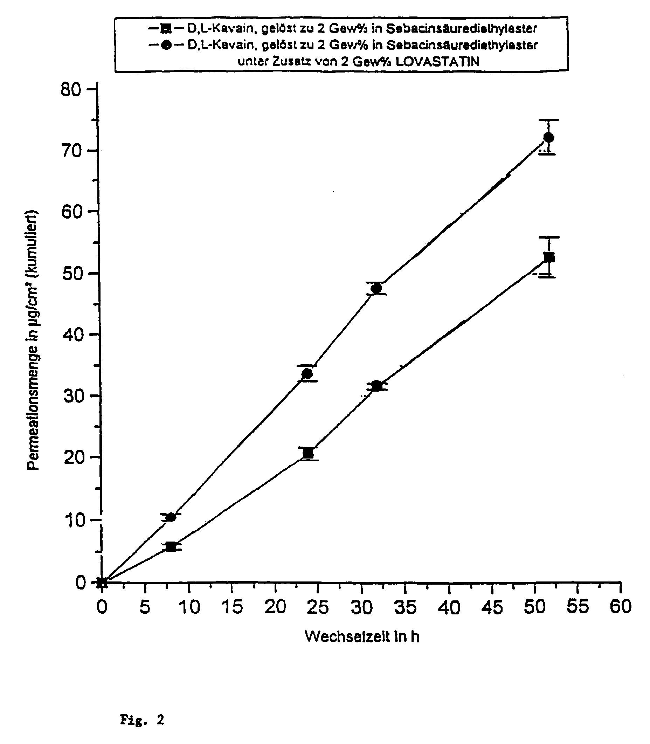

Die Erfindung wird anhand der in Fig. 1 und 2 dargestellten Messergebnisse verdeutlicht. Unter den normalerweise nur ungenügend resorbierbaren Wirkstoffen sind neben Morphin beispielhaft zu nennen: Theophyllin, L-Thyroxin, Ergotamin, D,L-Kavain, D,L-Warfarin.

Fig. 1 zeigt den Einfluß des Lipidsenkers LOVASTATIN auf die Permeationsrate von Morphin-Base an excidierter Meerschweinchenhaut
(freigesetzt in 0,9%iger Kochsalzlösung bei T=37°C, n=3, +/- SD)

Das Beispiel belegt, das durch den Zusatz des Lipidsenkers LOVASTATIN auch schon in geringen Mengen (2 Gew.-%) eine Steigerung der Permeationsrate um das Doppelte nach 24 h erzielt werden kann.
Eie Steigerung wird auch noch über diesen Zeitraum hinaus (bis 48 h) mit ca. 80% aufrechterhalten

Fig. 2 zeigt den Einfluß des Lipidsenkers LOVASTATIN auf die Permeationsrate von D,L-Kavain an excidierter Meerschweinchenhaut
(freigesetzt in isotonischem Phosphatpuffer pH 7,4 bei T=37°C, n=3, +/- SD)

Das Beispiel belegt, daß durch den Zusatz des Lipidsenkers LOVASTATIN auch schon in geringen Mengen (2 Gew.-%) eine Steigerung der Permeationsrate eines transdermal schlecht resorbierbaren Wirkstoffes um 70% nach 24 h erzielt werden kann.
Eine Steigerung wird auch noch über diesen Zeitraum hinaus (bis 52 h) mit 50% aufrechterhalten.

## Patentansprüche

1. Transdermales Therapeutisches System zur Applikation von wirkstoffen durch die Haut, enthaltend eine Steigerung der perkutanen Absorptionsrate von transdermal normalerweise nur ungenügend resorbierbaren Wirkstoffen bewirkende Zusätze, dadurch gekennzeichnet, daß die Zusätze HMG-CoA-Reduktase-Inhibitoren sind.

2. Zubereitung nach Anspruch 1, dadurch gekennzeichnet, daß die Zusätze LOVASTATIN, SIMVASTATIN, MEVASTATIN und PROVASTATIN sind.

3. Zubereitung nach Anspruch 1, dadurch gekennzeichnet, daß der Anteil der HMG-CoA-Reduktase-Inhibitoren mindestens 0,1, höchstens 20 Ma% beträgt.

## Claims

1. A transdermal therapeutic system for application of active substances through the skin comprising additives causing an increase in the percutaneous absorption rate of active substances which can normally only insufficiently be absorbed transdermally, characterized in that the additives are HMG-CoA-reductase-inhibitors.

2. The preparation according to claim 1 characterized in that the additives are LOVASTATIN, SIMVASTATIN, MEVASTATIN, and PROVASTATIN.

3. The preparation according to claim 1 characterized in that the portion of the HMG-CoA-reductase-inhibitors amounts to at least 0.1 and a maximum of 20%-wt.

## Revendications

1. Système thérapeutique transdermique pour l'application de principes actifs à travers la peau, contenant des additifs ayant pour effet d'augmenter le taux d'absorption percutanée de principes actifs manifestant normalement une aptitude à la résorption seulement insuffisante par voie transdermique, caractérisé en ce que les additifs sont des inhibiteurs de la HMG-CoA-réductase.

2. Formulation selon la revendication 1, caractérisée en ce que les additifs sont la LOVASTATINE, la SIMVASTATINE, la MÉVASTATINE et la PROVASTATINE.

3. Formulation selon la revendication 1, caractérisée en ce que la fraction des inhibiteurs de la HMG-CoA-réductase représente au moins 0,1, au maximum 20 Ma%.
